# EUROPEAN PATENT APPLICATION

(11) **EP 1 241 178 A1**
(43) Date of publication of application: **18.09.2002**
(21) Application number: 00980004.6
(22) Date of filing: 08.12.2000
(51) Int. Cl.: C07K 14/435, C07K 1/30, A61K 38/17, A61P 43/00, A61P 17/02, A61K 7/00

(54) **PROCESS FOR PRODUCING FUNCTIONAL SILK FIBROIN AND UTILIZATION OF THE SAME**

(30) Priority: 09.12.1999 JP 34998199
(71) Applicant: National Institute of Agrobiological Sciences, Tsukuba-shi, Ibaraki 305-8602 (JP); Kowa Co., Ltd., Naka-ku, Nagoya-shi, Aichi 460-0003 (JP)
(72) Inventor: TSUBOUCHI, Kozo, Kitasoma-gun, Ibaraki 302-0102 (JP); YAMADA, Hiroo, Tsukuba-shi, Ibaraki 305-0051 (JP); TAKASU, Yoko, Tsukuba-shi, Ibaraki 305-0821 (JP); NAKAO, Hiroshi, Tsuchiura-shi, Ibaraki 300-0813 (JP)
(74) Representative: Hartz, Nikolai F., Dr.
(86) International application number: JP0008697
(87) International publication number: WO01042300

(57) **Abstract**

Provided is a process for producing undegraded silk fibroin, which comprises dissolving a cocoon layer or cocoon yarn of fresh, dry or cooked cocoons, raw silk, or silk fabrics, or residual yarns thereof in an aqueous solution of a neutral salt, and then subjecting the resulting solution to fractional precipitation to separate silk fibroin from silk sericin; subjecting the resulting solution to the following step a) or b):
a): treatment with an alkaline aqueous solution under mild conditions,
b) treatment with an aqueous urea solution under mild conditions, thereby removing silk sericin; or subjecting the resulting solution to treatment with pressurized hot water or enzymatic degumming.

The present invention makes it possible to produce undegraded silk fibroin (molecular weight: 350000 to 370000) with industrial advantage comparable in function to that obtained from liquid silk. From the thus-obtained silk fibroin, excellent additives for cell culture, wound healing promoters and cosmetic compositions.

## Description

### Technical Field

The present invention relates to a process for producing undegraded silk fibroin having excellent cell proliferation promoting action; and its application in medical or cosmetic fields.

### Background Art

Silk fibroin has conventionally been studied as a wound dressing material in the powdery or film form, because it has healing promoting action of the skin tissue lost by an injury, burn or the like (Japanese Patent Laid-Open No. Hei 1-254164, Hei 8-198970, Hei 9-192210, or Hei 11-104228). Such action also permits use of silk fibroin as a cosmetic composition.

The healing promoting action of a wound dressing material making use of the conventional silk fibroin is however not fully satisfactory and there is a demand for the provision of a more effective wound healing promoter.

An object of the present invention is therefore to provide a process for acquiring a naturally occurring component excellent in regenerating capacity of lost skin tissue, in other words, excellent in cell proliferation promoting effect and its use.

### Disclosure of the Invention

The present inventors have therefore carried out various investigations, paying attention to the conventional production process of silk fibroin and found that in the conventional silk yarn producing process, silk fibroin has been degraded and has a reduced molecular weight.

It has been revealed for the first time by the present inventors as a result of analysis using electrophoresis that in the conventional silk yarn producing process composed of fresh cocoons - cocoon drying step - cocoon cooking step - reeling step - raw silk - degumming step - silk yarn, fibroin has been slightly reduced in molecular weight by the step of raw silk, though the degree of reduction varies depending on the storage conditions of the fresh cocoons or dry cocoons, or temperature or time of each step; and that in the subsequent step, its reduction proceeds further owing to degradation.

It has been found that such degraded fibroin has almost no cell proliferation promoting effect, while undegraded silk fibroin available under special conditions from cocoon yarn, raw silk or silk yarn has selectively potent cell proliferation promoting effect and use of it makes it possible to produce highly effective and safe wound healing promoters and cosmetic compositions, leading to the completion of the present invention.

In the present invention, there is thus provided undegraded silk fibroin available from the cocoon layer or cocoon yarn of fresh, dry or cooked cocoons, raw silk, or silk fabrics, or residual yarns thereof.

In the present invention, there is also provided a process for producing undegraded silk fibroin, which comprises dissolving the cocoon layer or cocoon yarn of fresh, dry or cooked cocoons, raw silk, or silk fabrics, or residual yarns thereof in an aqueous solution of a neutral salt, and subjecting the resulting solution to fractional precipitation, thereby separating silk fibroin from silk sericin.

In the present invention, there is also provided a process for producing undegraded silk fibroin, which comprises degumming the cocoon layer or cocoon yarn of fresh, dry or cooked cocoons, raw silk, or silk fabrics, or residual yarns thereof by subjecting it (them) to the following step a) or b):
a) treatment with an aqueous alkali solution under mild conditions, or
b) treatment with an aqueous urea solution under mild conditions.

In the present invention, there is also provided a process for producing undegraded silk fibroin, which comprises enzymatically degumming the cocoon layer or cocoon yarn of fresh, dry or cooked cocoons, raw silk, or silk fabrics, or residual yarns thereof.

In the present invention, there are also provided a cell proliferation promoter and a wound healing promoter, each comprising as an effective ingredient the undegraded silk fibroin available by any one of the above-described processes.

In the present invention, there is also provided a cosmetic composition comprising the undegraded silk fibroin available by any one of the above-described processes.

### Brief Description of the Drawings

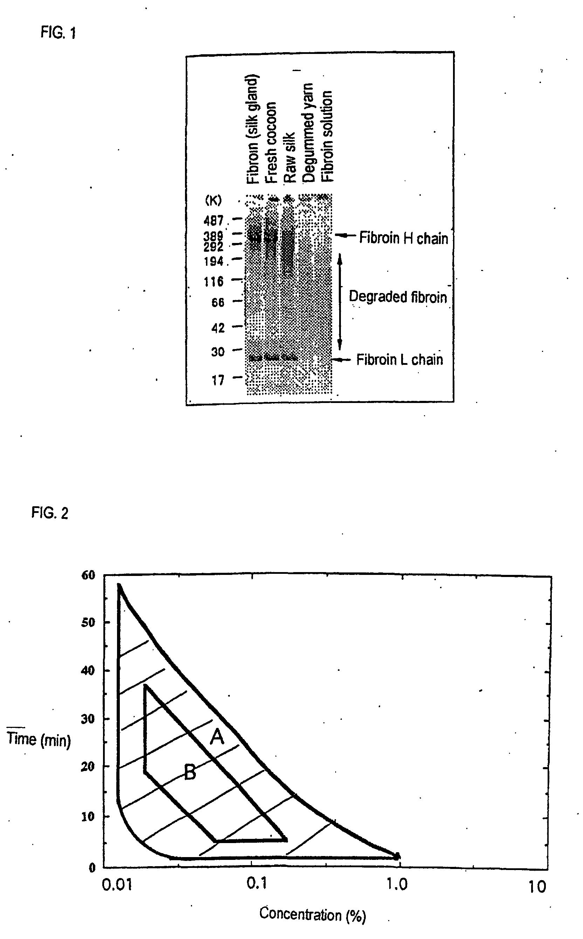

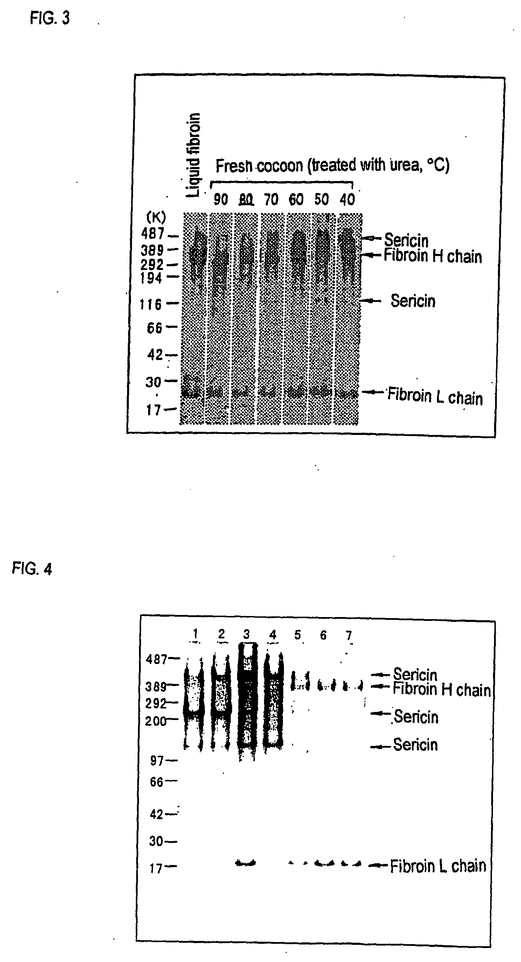

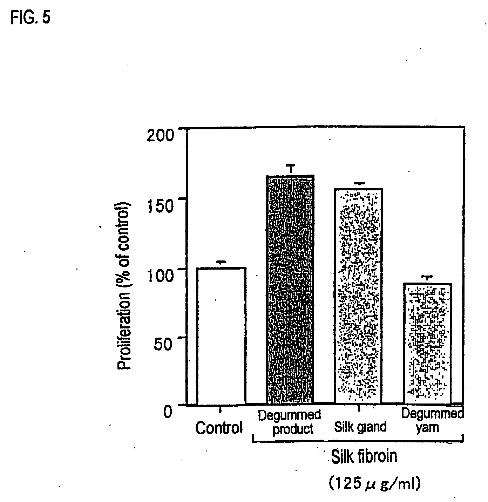

FIG. 1 is an electrophoretogram illustrating the degraded state of silk fibroin of each material in Example 1; FIG. 2 illustrates a remaining range of the undegraded silk fibroin in a boiling aqueous solution of sodium carbonate; FIG. 3 is an electrophoretogram illustrating the degraded condition of silk fibroin treated with an aqueous urea solution; FIG. 4 is an electrophoretogram illustrating the fractionated state of silk fibroin after treatment with an aqueous solution of a neural salt and fractional precipitation; and FIG. 5 illustrates effects of silk fibroin on proliferation of fibroblast.

### Best Mode for Carrying out the Invention

A silkworm secretes silk in the liquid form into its silk gland lumen and this silk is called "liquid silk". Liquid silk is composed of silk fibroin and silk sericin. Liquid silk fibroin has a molecular weight of about 370000 (Tasiro Yutaka and Otsuki Eiichi, Journal of Cell Biology, Vol. 46, Pl(1970)). Silk fibroin having a molecular weight of about 370000 is divided by reduction into a portion of about 350000 in molecular weight and another portion of about 25000 in molecular weight. Here, the silk fibroin having a molecular weight of about 350000 or 370000 is called "undegraded silk fibroin".

Upon building a cocoon, a silkworm spins liquid silk, and forms a cocoon (made of cocoon yarn and pupa). Silk fibroin exists in the central part of cocoon yarn, while silk sericin exists therearound. The ratio is known to be 70 to 80 (fibroin) / 20 to 30 (sericin). Raw silk is formed of several or several tens of cocoon yarns collected (reeling). A step of removing silk sericin from cocoon yarn, raw silk or raw silk fabric is called "degumming" and fiber after degumming is called "silk yarn" or "silk yarn fibroin". Silk sericin is not always removed completely by degumming. Sometimes, 50% degumming or 70% degumming is employed for removal of about half or 70% of sericin and such a fiber is also called "silk yarn" because it has been degummed. The term "raw silk fabric" means silk fabric which has not been degummed. In general, the term "silk" embraces fibroin itself, sericin itself and mixture of fibroin and sericin. Either of fibroin or sericin in any form such as film, powder or fiber is called silk. The terms "silk fibroin" and "fibroin", or the terms "silk sericin" and "sericin" have the same meanings. The term "silk fibroin" embraces "silk fibroin film" "silk fibroin powder" and "silk yarn fibroin" and of these, the term "silk yarn fibroin" means fibers obtained by degumming cocoon yarn, raw silk, or raw silk fabric, or residual yarn thereof.

The conventional production process of silk yarn is as follows: after obtaining raw silk or raw silk fabric by drying fresh cocoons (fresh cocoons: cocoons free from treatment for storage) which have been produced by silkworm farmers, cooking and then reeling, the resulting raw silk or raw silk fabric is degummed, whereby a silk yarn or silk fabric is obtained. The waste yarn from these steps is called "residual yarn". Cocoon drying is carried out by gradually decreasing the temperature from 115 to 120°C to about 80°C over 5 to 6 hours. Upon cocoon cooking, cocoon is treated with steam or hot water of 100 to 105°C for about 10 minutes. Examples of the conventional degumming process include boiling in an aqueous solution containing an alkaline sodium salt such as sodium carbonate or sodium bicarbonate or soap (most usual process), degumming by immersing in pressurized hot water (ex. hot water of 120°C), and enzymatic degumming. It was considered that in such a conventional silk yarn production step, silk yarn fibroin had not been degraded. The study by the present inventors, however, has revealed that silk fibroin has been degraded in the conventional silk yarn production process. Described specifically, silk fibroin of a cell culturing bed or wound dressing agent using the conventional silk yarn as a raw material has been degraded and undegraded silk fibroin has not remained.

Silk fibroin is also available from liquid silk. Undegraded silk fibroin can be obtained, for example, by taking out the gel-like content (liquid silk) from the middle section and posterior section of the silk gland of a silkworm and removing a small amount of silk sericin adhered to the content by using an aqueous solution or the like. This process however needs extraction of the silk gland from the dissected body of a silkworm and then extraction of silk fibroin from the silk gland lumen. The amount of silk fibroin available from one silkworm is about 0.4 g at the maximum. This process is not suited for industrial production, because silk fibroin thus obtained tends to contain impurities such as silkworm humor and silk gland cells and it takes time and labor for obtaining silk fibroin.

In the present invention, the industrially advantageous cocoon layer or cocoon yarn of fresh, dry or cooked cocoons, raw silk, or silk fabrics, or residual yarn thereof is used as a material. Silk fibroin is apt to be degraded during storage so that use of fresh cocoons as a raw material is most preferred. It is preferred to store fresh cocoons, dry cocoons, raw silk, silk yarn or residual yarn thereof at a temperature not greater than room temperature while shielding it from light. Prior to use, it is necessary to study whether undegraded silk fibroin remains or not.

When an aqueous solution of a neutral salt is used, the above-described raw material is dissolved in the aqueous solution of a neutral salt, followed by fractional precipitation. Examples of the neutral salt include lithium thiocyanate, calcium chloride, lithium bromide and sodium thiocyanate. Although the concentration of the neutral salt varies depending on the kind of the neutral salt, a saturated aqueous solution or a concentration of 50% saturation or greater is preferred in any neutral salt. Particularly, concentrations of 80% saturation or greater are preferred. When lithium thiocyanate is used, its saturated aqueous solution has about 80% (weight (g)/volume (mL)) concentration. This will hereinafter be described "%" simply. The aqueous solution of the above-described neutral salt has a pH of 5 to 8.

Upon fractional precipitation after a solution of the raw material is prepared, acetone or alcohol, particularly, ethanol is preferably added to crystallize amorphous silk fibroin. Precipitates are preferably collected while adding ethanol successively.

The mild treatment conditions with an aqueous alkali solution in the step a) are conditions of pH, temperature and time selected so as not to degrade silk fibroin. Treatment is preferably conducted under conditions changed as needed within the following range: in an aqueous alkali solution having a pH 10 to 11.5 at a boiling temperature under atmospheric pressure for a time of from 2 to 60 minutes. At pHs less than 10, degumming is not sufficient, while at pHs exceeding 11.5, the degradation rate of silk fibroin becomes uncontrollably high. A preferable pH range of the aqueous solution is from 10.5 to 11.5. The aqueous alkali solution usable for this treatment is an aqueous solution of an alkaline sodium salt such as sodium carbonate, sodium bicarbonate, sodium silicate, sodium metasilicate, sodium phosphate or sodium hydroxide. In alkali degumming, an aqueous solution of sodium carbonate is especially preferred because it has a sufficient buffer effect.

In order to obtain undegraded silk fibroin by degumming without degrading silk, it is necessary to control, as needed, treatment conditions such as pH, temperature and time upon degumming. For example, treatment at a temperature (ex. 110 or 120°C) of boiling point or higher needs pH close to neutral or short treatment time. Treatment at a temperature not higher than boiling point needs high pH or longer treatment time. Thus, the conditions are changed as needed. It is needless to say that when a sodium salt other than sodium carbonate is used, conditions are changed as needed with reference to those for sodium carbonate. In the treatment in this step a), immersion of the raw material in an aqueous alkali solution is indispensable and during immersion, stirring may be conducted.

The mild conditions in an aqueous urea solution in the step b) are conditions of temperature and time selected so as not to cause degradation of silk fibroin. From the viewpoints of prevention of degradation of silk fibroin, reaction efficiency and industrial operability, treatment with a 30% or greater aqueous urea solution at 70 to 90°C for 60 to 180 minutes is preferred. Treatment with a 45% or greater aqueous urea solution at 75 to 85°C for 90 to 150 minutes is more preferred. The aqueous urea solution may be added with mercaptoethanol or the like.

It is needless to say that similar to the step a), temperature, concentration and time upon urea degumming can be combined as needed to set proper treatment conditions. In the treatment in step b), immersion of the above-described material in an aqueous urea solution is indispensable and this treatment may be conducted while stirring.

Enzymatic degumming is a method of applying protease to degumming of raw silk or fresh cocoons. Instead of papain which was employed frequently, Alcalase (Koshindo Kagaku Kogyosho) has recently been employed as an enzyme. Enzymatic degumming by Alcalase needs pre-treatment, more specifically, pre-treatment at pH 9 to 10, preferably pH 9.0 to 9.6, within 10 minutes, preferably 5 minutes at 80°C or within 60 minutes, preferably 10 minutes at 60°C. Then, main treatment is conducted by adding Alcalase and degumming at 50 to 60°C. The degumming is preferably conducted within 60 minutes, especially within 20 minutes. In this case, the cocoon layer is preferably raveled out as finely as possible. During this raveling-out work, stirring may be conducted.

The fractional precipitate (undegraded silk fibroin) obtained using an aqueous solution of a neutral salt, the undegraded silk fibroin available by the step a) or b), or the undegraded silk fibroin available by treatment with pressurized hot water or by enzymatic degumming is dissolved in a solution containing a solubilizing agent, followed by dialysis for demineralization, whereby an aqueous solution of the undegraded silk fibroin can be obtained. Examples of the solubilizing agent include lithium thiocyanate, calcium chloride, lithium bromide, sodium thiocyanate, ethanol and so on.

Degraded silk fibroin hardly exhibits cell proliferation promoting action, while the undegraded silk fibroin thus obtained exhibits excellent cell proliferation promoting action. It is therefore called "functional silk fibroin" and is useful for various additives for cell culture (especially additives for animal cells including human cells), and wound healing promoters and cosmetic compositions (which may be called "preparations for external use", collectively)

Upon use as a wound healing promoter or cosmetic composition, the undegraded silk fibroin (functional silk fibroin) in the form of a hydrogel, film, powder or the like is preferred as a preparation for external use which can be applied to the wound or skin. The undegraded silk fibroin in the film form is available by casting an aqueous solution of the undegraded silk fibroin on a flat plate and then drying. That in the powdery form is available by pulverizing the above-described film; air-spraying an aqueous solution of the undegraded silk fibroin; or washing, drying and pulverizing a precipitate obtained by stirring an aqueous solution of the undegraded silk fibroin or by adding an alcohol to an aqueous solution of the undegraded silk fibroin.

The undegraded silk fibroin can also be used in the form of an ordinary preparation for external use such as ointment, cream, cataplasm and so on. Such ointment, cream or cataplasm is available only by incorporating the undegraded silk fibroin in the ordinarily employed ointment base, cream base or cataplasm base.

The undegraded silk fibroin is added to such a preparation for external use in an amount of 0.001 to 30%, especially 0.1 to 10%, though depending on the form or base.

### Examples

The present invention will hereinafter be described in further detail by Examples. It should however be borne in mind that the present invention is not limited to or by them.

### Example 1 (confirmation of degradation of silk fibroin in a silk yarn production step)

As samples, employed were the cocoon layer of fresh cocoons within one month after cocoon building, the cocoon layer of dry cocoons, the cocoon layer of cooked cocoons (standard cocoons cooked by a cocoon cooking machine for cocoon test), raw silk and a silk yarn obtained by boiling the raw silk in 50 times the weight (bath ratio: 50 times) of a 0.05% aqueous solution of sodium carbonate for one hour at atmospheric pressure. Each of the samples was dissolved in an about 80% aqueous solution of lithium thiocyanate (pH 7). The solution was dialyzed against a 30% aqueous urea solution, followed by analysis, by SDS polyacrylamide gel (2-15% gradient gel) electrophoresis, of silk fibroin in the solution added with a 4% 2-mercaptoethanol solution and the solution free of it. Incidentally, the dry cocoons, cooked cocoons, raw silk and silk yarn were each prepared in a conventional manner. As a result, from the solution added with a 2-mercaptoethanol solution, a band of silk fibroin corresponding to about 350000 was recognized clearly. From the dry cocoons, a band of silk fibroin corresponding to about 350000 was recognized clearly, but the band was a little pale. It became paler in the cooked cocoon and raw silk, suggesting that undegraded silk fibroin remained but degraded silk fibroin also existed. From the silk yarn, no band of silk fibroin corresponding to about 350000 was recognized. When the solution was not added with a 2-mercaptoethanol solution, a band of silk fibroin corresponding to about 370000 appeared in the cocoon layer of the fresh cocoons, dry cocoons and cooked cocoons, and raw silk, but not in the silk yarn. Electrophoresis for confirming existence of undegraded silk fibroin gives the same result whether a band of about 350000 or about 370000 is used. Use of fresh cocoons is preferred in order to obtain undegraded silk fibroin, but dry cocoons, cocked cocoons or raw silk is also usable (refer to FIG. 1).

### Example 2

The cocoon layer of fresh cocoons was thoroughly raveled out, followed by immersion in a boiling 0.05% aqueous solution of sodium carbonate (bath ratio: 50 times) in the atmospheric pressure. After immersion for each of 5, 10, 15, 20, 30, 40 and 60 minutes, the sample was washed with water and dried. After dissolution and dialysis in a similar manner to Example 1, degradation degree of silk fibroin was analyzed by electrophoresis. As a result, the longer the immersion time, the paler the band of silk fibroin corresponding to about 350000 gradually became. By immersion for 40 minutes, the band was slightly confirmed. The band was not confirmed as a result of immersion for 60 minutes. The gravimetric measurement before and after immersion of the cocoon layer in an aqueous sodium carbonate solution indicates that immersion for 5 minutes, 10 minutes and 60 minutes caused a weight reduction of 21.5%, 24.5% and 25.1%, respectively and silk sericin was almost removed by immersion for about 10 minutes. Removal of silk sericin depends on the degree of raveling-out of the cocoon layer or bath ratio.

### Example 3

In a similar manner to Example 2 except the concentration of sodium carbonate and immersion time therein were changed, a test was conducted while boiling at atmospheric pressure. The results are shown in FIG. 2. Undegraded silk fibroin remains within a range of A (2 to 60 minutes at a concentration of 0.012 to 1.0%), preferably within a range of B (5 to 40 minutes at a concentration of 0.02 to 0.2%) in FIG. 2.

### Example 4

After 50 mg of fresh cocoons was immersed in a solution containing 48% urea and 4% 2-mercaptoethanol, it was treated for 2 hours at each of various temperatures (40, 50, 60, 70, 80 and 90°C). The thus-treated cocoons were washed with water, dissolved in an about 80% aqueous solution of lithium thiocyanate, demineralized and subjected to SDS polyacrylamide gel electrophoresis. The result. (FIG. 3) suggests that sericin was not removed sufficiently at a treating temperature not greater than 70°C, while silk fibroin was easily degraded at 90°C; and that treatment at 80°C did not degrade silk fibroin but removed silk sericin.

### Example 5

An about 5% silk solution was obtained by dissolving the cocoon layer of fresh cocoons in an about 80% aqueous solution of lithium thiocyanate (pH 7). Ethanol was added successively as shown in Table 1 and silk precipitates were collected. Silk fibroin of each of them was analyzed by SDS polyacrylamide gel electrophoresis. The results are shown in FIG. 4. From the precipitates obtained in Nos. 5, 6 and 7 in Table 1, a band of silk fibroin corresponding to about 350000 was observed. The precipitates available at an ethanol concentration not greater than about 74% were removed and precipitates available at the ethanol concentrations of 74% or greater, preferably of about 80% or greater but not greater than 90% can be used as undegraded silk fibroin.

**Table 1**

| Lane NO. | Treatment |
|---|---|
| 1 | Precipitate formed by the addition of 80 mL of ethanol to 50 mL of the supernatant |
| 2 | Precipitate formed by the addition of 20 mL of ethanol to the supernatant of Lane No. 1 |
| 3 | Precipitate formed by the addition of 20 mL of ethanol to the supernatant of Lane No. 2 |
| 4 | Precipitate formed by the addition of 20 mL of ethanol to the supernatant of Lane No. 3 |
| 5 | Precipitate formed by the addition of 60 mL of ethanol to the supernatant of Lane No. 4 |
| 6 | Precipitate formed by the addition of 20 mL of ethanol to the supernatant of Lane No. 5 |
| 7 | Precipitate formed by allowing the supernatant of Lane No. 6 to stand overnight |

### Example 6

The cocoon layer of fresh cocoons and raw silk obtained from these fresh cocoons in a conventional manner were enzymatically degummed. The cocoon layer was raveled out well. The cocoon layer and raw silk were subjected to treatment with "Rahzen Power II" (trade name; product of Koshindo Kagaku Kogyosho) prior to degumming. First, each of them was treated with an aqueous solution of "Rahzen Power II" (pH 9.7) at 60°C for 10 minutes (Cocoon layer - 1, Raw silk - 1) or for 70 minutes (Cocoon-layer - 2, Raw silk - 2). The solution was then diluted to 1/2, followed by the addition of 2.5L of Alcalase and "Rahzen Power" in an amount of 1% of the amount of the yarn. For 15 minutes (Cocoon layer - 1, Raw silk - 1) or for 40 minutes (Cocoon layer - 2, Raw silk - 2) at 55°C, degumming was conducted at a bath ratio of 25 times. The degradation degree of silk fibroin of them was studied by electrophoresis as in Example 1. The results have indicated that a band of undegraded silk fibroin was confirmed in Cocoon layer - 1, but the band was not apparent in another sample.

Each sample exhibited a degumming loss ratio of 23% or greater, suggesting that sericin was almost removed.

### Example 7

The cocoon layer (which had been raveled out sufficiently) of fresh cocoons and raw silk obtained in a conventional manner were degummed with pressurized hot water (100°C or greater). The temperature and time were set at 105°C, 110°C and 120°C and 10 minutes, 30 minutes and 60 minutes, respectively. Whether undegraded silk fibroin remained after degumming or not was studied by electrophoresis and remaining in the raw silk was not recognized. In the cocoon layer treated at 105°C for 10 minutes (degumming loss ratio: 15%) and at 110°C for 10 minutes (degumming loss ratio: 20%), existence of undegraded silk fibroin was confirmed, but from another sample, existence was not confirmed clearly.

In pressurized hot water degumming at 100°C or greater, temperature is preferably set at 115°C or less. Degumming is conducted within 20 minutes, preferably within 10 minutes, when the temperature is set at 110°C.

### Example 8

The dry undegraded silk fibroin obtained in each of Examples 2 to 5 was dissolved in an about 80% aqueous solution of lithium thiocyanate, followed by dialysis against water, whereby an aqueous solution of the undegraded silk fibroin was obtained. The resulting solution was cast on a flat plate and dried to obtain a film. The film was pulverized into powder. On the other hand, another powder was obtained by air-spraying of the aqueous solution of the undegraded silk fibroin. This aqueous solution of the undegraded silk fibroin was stirred or added with an alcohol to obtain a precipitate, followed by washing with water, drying and pulverization, whereby undegraded silk fibroin powder was obtained.

### Example 9

Fibroblast derived from the human skin was seeded in a 24-well plate. After culturing for 24 hours, the culture medium was changed. Three fibroins, that is, liquid silk fibroin from silk gland, the undegraded silk fibroin derived from fresh cocoons obtained by treatment for 2 hours in an aqueous urea solution of 80°C in Example 4 and silk fibroin derived from raw silk were each added to the medium at a concentration of 125 µg/mL. Forty eight hours later, MTT (oxidation-reduction indicator for measuring respiration of cell) was added and the mixture was incubated for 4 hours. The viable count was compared with the silk fibroin free group. As illustrated in FIG. 5, the liquid silk fibroin (silk gland) and silk fibroin derived from fresh cocoons (degummed product) exhibited the same level of proliferation promoting action, but no promoting action was recognized in the conventional silk fibroin derived from raw silk (degummed yarn). Silk fibroin precipitates obtained in Nos. 5, 6 and 7 of Example 5 shown in Table 1 were found to exhibit cell proliferation promoting action at least comparable to the degummed product of FIG. 5.

### Industrial Applicability

The present invention makes it possible to advantageously produce undegraded silk fibroin (molecular weight: 350000 to 370000) comparable to that available from liquid silk. By the use of it, excellent additives for cell culture, wound healing promoters and cosmetic compositions are available.

## Claims

1. Undegraded silk fibroin obtained by degumming a cocoon layer or cocoon yarn of fresh, dry or cooked cocoons, raw silk, or silk fabrics or residual yarns thereof.

2. A process for producing undegraded silk fibroin, which comprises dissolving the cocoon layer or cocoon yarn of fresh, dry or cooked cocoons, raw silk, or silk fabrics or residual yarns thereof in an aqueous solution of a neutral salt, and subjecting the resulting solution to fractional precipitation, thereby separating silk fibroin from silk sericin.

3. A process for producing undegraded silk fibroin, which comprises subjecting the cocoon layer or cocoon yarn of fresh, dry or cooked cocoons, raw silk, or silk fabrics or residual yarns thereof to the following step a) or b):
a) treatment with an aqueous alkali solution under mild conditions, or
b) treatment with an aqueous urea solution under mild conditions, thereby degumming the same.

4. A process for producing undegraded silk fibroin, which comprises enzymatically degumming the cocoon layer or cocoon yarn of fresh, dry or cooked cocoons, raw silk, or silk fabrics, or residual yarns thereof.

5. A cell proliferation promoter, which comprises, as an effective ingredient, the undegraded silk fibroin obtained by the production process of Claim 2, 3 or 4.

6. A wound healing promoter, which comprises the undegraded silk fibroin obtained by the production process of Claim 2, 3 or 4.

7. A cosmetic composition, which comprises the undegraded silk fibroin obtained by the production process of Claim 2, 3 or 4.
